Europäisches Patentamt

European Patent Office

Office européen des brevets

(11) Veröffentlichungsnummer: **0 277 934 B1**

## EUROPÄISCHE PATENTSCHRIFT

(45) Veröffentlichungstag der Patentschrift: **04.08.93**

(51) Int. Cl.5: **C12P 19/20**

(21) Anmeldenummer: **88890011.5**

(22) Anmeldetag: **25.01.88**

(54) **Verfahren zur Herstellung von Zuckersirupen durch enzymatische Direktverzuckerung von stärkereichen Rohstoffen, insbesondere frischen oder getrockneten Cassavawurzeln.**

(30) Priorität: **29.01.87 AT 178/87**

(43) Veröffentlichungstag der Anmeldung:
**10.08.88 Patentblatt 88/32**

(45) Bekanntmachung des Hinweises auf die Patenterteilung:
**04.08.93 Patentblatt 93/31**

(84) Benannte Vertragsstaaten:
**DE FR GB NL SE**

(56) Entgegenhaltungen:
**EP-A- 0 068 043**
**GB-A- 1 181 174**

**CHEMICAL ABSTRACTS, Band 99, Nr. 1, 4. Juli 1983, Seite 388, Zusammenfassung Nr. 4021z, Columbus, Ohio, US; M. KUMAKURA et al.: "Pretreatment of starch raw materials and their enzymic hydrolysis by immobilized glucoamylase"**

**DIE STÄRKE, Band 6, 1954, Seiten 122-124, Wissenschaftliche Verlagsgesellschaft mbH, Stuttgart, DE; L. DOSTAL: "Laboratoriumsversuche zur Gewinnung von Stärkesirup unmittelbar aus Mais-, Milo- und**

**Maniokmehl"**

(73) Patentinhaber: **VOGELBUSCH GESELLSCHAFT m.b.H.**
**Blechturmgasse 11**
**A-1050 Wien(AT)**

(72) Erfinder: **Sarhaddar, Schahroch, Dipl.-Ing.**
**Barawitzkagasse 27/2/1**
**A-1190 Wien(AT)**
Erfinder: **Berghofer, Emmerich, Dipl.-Ing.Dr.**
**Guggenbergerstrasse 14**
**A-3021 Pressbaum(AT)**

(74) Vertreter: **Wolfram, Gustav, Dipl.-Ing.**
**Patentanwälte Sonn, Pawloy, Weinzinger & Wolfram, Riemergasse 14**
**A-1010 Wien (AT)**

## Beschreibung

Die Erfindung betrifft ein Verfahren zur Herstellung von Zuckersirupen aus stärkehältigen Rohstoffen, insbesondere Cassava (Maniok).

Cassava oder Maniok (Manihot esculenta, Crantz) steht an sechster Stelle der bedeutendsten Nahrungsmittelpflanzen der Erde. Die Weltproduktion wird auf über 100 Mio Tonnen geschätzt. Ihr Anbau wird in etwa 80 Ländern auf der ganzen Welt in einer Zone von 30° nördlich und südlich des Äquators betrieben. Die Cassavapflanze stellt nur sehr geringe Ansprüche an die Bodenqualität. Durch ihre extreme Anpassungsfähigkeit sowie ihre hohe Produktivität gewinnt sie immer mehr an Bedeutung. Ungefähr 80 % der Welternte werden direkt für den menschlichen Verzehr verwendet. Die restlichen 20 % werden entweder industriell zur Gewinnung von Tapiokastärke - so wird die aus Cassava isolierte Stärke bezeichnet - oder zu Alkohol weiterverarbeitet oder in getrockneter Form (Chips oder Pellets) als Tierfutter verbraucht. Nur ein sehr kleiner Teil wird gegenwärtig zur Produktion von Zuckersirupen für Nahrungszwecke eingesetzt. Gerade dieser Bereich wird aber in Zukunft vermehrte Bedeutung erlangen. Der Pro-Kopf-Verbrauch an Zucker liegt nämlich in den unterentwickelten, cassavaproduzierenden Ländern derzeit nur bei 9 bis 13 kg/Jahr im Gegensatz zu 40 bis 50 kg/Jahr in den industrialisierten Ländern. Während in den letzteren Ländern der Verbrauch stagniert oder sogar leicht zurückgeht, ist in den übrigen Regionen eine steigende Tendenz erkennbar. Neben Zuckerrohr werden die unterentwickelten Länder in Zukunft mit Sicherheit in vermehrtem Ausmaß einheimische, stärkereiche Rohstoffe, wie z.B. Cassava, zur Deckung ihres Bedarfes an niedermolekularen Kohlehydraten heranziehen.

Gegenwärtig erfolgt die Herstellung von Zuckersirupen, wie Glucosesirup und fruktosehältigen Isosirupen, aus Cassava in der Weise, daß vorerst die Stärke aus den Wurzeln isoliert wird. Da Tapiokastärke ein sehr hochgereinigtes Produkt darstellt und sich in seiner Zusammensetzung von anderen, isolierten Stärkearten nur unwesentlich unterscheidet, erfolgt ihre Verflüssigung, Verzuckerung und Isomerisierung nach den gleichen Verfahren und mit gleich gutem Erfolg, wie die von Mais-, Weizen- oder Kartoffelstärke.

Es gibt schon lange die Überlegung, daß es umständlich ist, in aufwendigen Trennschritten zunächst die Stärke aus den Rohstoffen zu isolieren und diese dann erst zu hydrolysieren und zu isomerisieren. Einfacher wäre es, die Stärke unmittelbar in den stärkehältigen Rohstoffen zu hydrolysieren und die entstehende Kohlenhydratlösung erst danach zu reinigen. Mit der großtechnischen Einführung der sehr substratspezifisch wirkenden, stärkeabbauenden Enzyme konnte diese Verfahrensweise dann auch tatsächlich beim Rohstoff Mais erfolgreich in die Praxis umgesetzt werden (TEGGE und SEILER, 1972; BOS und NORR, 1974).

Es hat nicht an Versuchen gefehlt, die Direkthydrolyse auch bei anderen stärkereichen Rohstoffen anzuwenden. Bei Kartoffeln waren aber nach TEGGE und LAMBERTY (1975) diese Versuche nicht sehr erfolgversprechend. Neben einer ungenügenden Ausbeute bereitete vor allem die Raffination der Hydrolysate - bedingt durch die spezifische Zusammensetzung des Kartoffelproteins - Schwierigkeiten.

Für Cassava wird zwar eine Direkthydrolyse zur Alkoholgewinnung beschrieben (MENEZES, 1978; LAGES und TANNENBAUM, 1978), es existieren aber hier keine Angaben und Verfahren zur Direktverzuckerung von Cassava für eine Zuckersirupproduktion und die Reinigung solcher Hydrolysate.

In der GB-A - 1 181 174 wird ein Direkthydrolyseverfahren zur Verzuckerung von stärkehaltigen Rohstoffen beschrieben, in dem allerdings der Aufschluß durch intermittierende Druckstöße während des Erhitzens in einem speziellen Röhrenkonverter erfolgt.

Die vorliegende Erfindung stellt sich die Aufgabe, ein Verfahren bereitzustellen, welches eine wirtschaftliche Direkthydrolyse von stärkehaltigen Rohstoffen, vor allem von frischen oder getrockneten Cassavawurzeln, und die Weiterverarbeitung der erhaltenen Hydrolysate zur Zuckersirupen gestattet.

Diese Aufgabe wird erfindungsgemäß dadurch gelöst, daß

a) aus zerkleinertem Rohstoff wasserlösliche Inhaltsstoffe ausgewaschen werden;

b) die im Rohstoff enthaltene Stärke ohne Isolierung unter schonenden Bedingungen, d.h. bei Temperaturen unter 100°C, vorzugsweise bei 85 - 95°C, und ohne weiteren mechanischen Aufschluß, und bei Gehalten an Trockensubstanz von über 20 % kontinuierlich verkleistert, enzymatisch mit alpha-Amylase verflüssigt und danach mit Glucoamylase verzuckert wird;

c) die Feststoffe aus der verzuckerten Maische durch Zentrifugieren oder Filtrieren abgetrennt werden;

d) der gewonnene Saft durch Adsorptionsfiltration mit Aktivkohle gereinigt und

e) durch Behandlung mit einer Kombination eines Kationenaustauschers und eines Anionenaustauschers entsalzt wird.

Als Rohstoff werden vorteilhaft frische Cassavawurzeln verwendet, welche vorzugsweise grob vorzerkleinert und anschließend naßfein zerkleinert werden.

Weiters werden vorteilhaft als Rohstoff getrocknete Cassavaprodukte in Form von Chips oder Pellets verwendet, die vorzugsweise grob trocken vorgemahlen und anschließend naßfein zerkleinert werden.

Nach einer bevorzugten Ausführungsform wird das bei der Feststoffabtrennung anfallende Waschwasser (Absüßwasser) als Verdünnungswasser beim Stärkeaufschluß eingesetzt.

Es ist zweckmäßig, daß zur Entsalzung des Saftes zuerst ein stark saurer Kationenaustauscher verwendet und daß der Saft nach Behandlung mit dem sauren Kationenaustauscher mit einem mittel- bis starkbasischen Anionenaustauscher behandelt wird.

Im einzelnen gestaltet sich das erfindungsgemäße Verfahren folgendermaßen:

Der erste Schritt, die Zerkleinerung der Rohstoffe, kann bei Verwendung frischer Wurzeln entweder mit Sägeblattreiben durchgeführt werden oder nach einer groben Vorzerkleinerung (z.B. mit einem Reibschnitzler) durch Naßfeinzerkleinerung mit geeigneten Mühlen (z.B. einer Zahnkolloidmühle).

Bei der Verarbeitung getrockneter Cassavaprodukte, wie Chips oder Pellets, kann die Zerkleinerung ebenfalls auf zwei Arten erfolgen: entweder durch eine alleinige Trockenvermahlung, z.B. mittels einer Hammer- oder Messermühle, auf eine Partikelgröße unter 1 mm oder durch grobe, trockene Vormahlung auf eine Partikelgröße unter 5 mm, gefolgt von einer Naßfeinzerkleinerung mit geeigneten Mühlen, z.B. einer Zahnkolloidmühle. Da eine alleinige Trockenvermahlung auf kleine Partikelgrößen unter 1 mm einen großen Energieaufwand erfordert, ist die zweistufige Vermahlung energetisch vorteilhafter und deshalb vorzuziehen.

Der nächste Verfahrensschritt des erfindungsgemäßen Verfahrens ist die Abtrennung der wasserlöslichen Inhaltsstoffe und des Fruchtwassers. Dazu werden die zerkleinerten Rohstoffe in Wasser suspendiert, die Feststoffe durch Zentrifugieren oder Filtrieren wieder abgetrennt und der Feststoffkuchen mit Wasser ausgewaschen. Durch diese Maßnahme werden bei frischen Wurzeln günstigerweise bereits bis zu 75 bis 85 %, bei Trockenproduktion 50 bis 60 % des Protein- und Ascheanteiles entfernt, wodurch sich die Wirtschaftlichkeit des erfindungsgemäßen Direkthydrolyseverfahrens stark verbessert. Es werden nämlich einerseits durch den verringerten Proteingehalt bei der nachfolgenden thermischen Verkleisterung und Verflüssigung der Stärke wesentlich weniger Farbstoffe durch Maillard-Reaktionen aus Protein und Zuckern gebildet; anderseits müssen bei der Reinigung der Hydrolysate mit Hilfe des relativ kostenintensiven Verfahrens der Aktivkohlefiltration und des Ionenaustausches auch weniger Farbstoffe, gelöstes Protein und Mineralstoffe entfernt werden.

Der ausgewaschene Rohstoff wird anschließend bei einer Temperatur unter 100°C, insbesondere bei 90° bis 95°C, kontinuierlich verkleistert, wobei zum Einmaischen des Rohstoffes das Waschwasser verwendet wird, welches beim späteren Absüßen des Feststoffkuchens nach der Verzuckerung anfällt.

Die thermische Verkleisterung und Verflüssigung der Stärke ist ein entscheidender Schritt für die wirtschaftliche Durchführung des erfindungsgemäßen Direkthydrolyseverfahrens zur Zuckersirupproduktion. Einerseits ist darauf Bedacht zu nehmen, daß sämtliche Stärke vollständig und rasch verkleistert und verflüssigt wird, anderseits soll die Farbstoffbildung durch Maillard-Reaktionen aus Protein und Zucker, sowie die Überführung von Zellwandbestandteilen in lösliche Form minimiert werden. Hierzu eignet sich z.B. das aus der EP 0 131 563 an sich bekannte Heißmaischverfahren, welches in einem Temperaturbereich unter 100°C bei kurzer Verweilzeit arbeitet.

Nach Durchführung der Verzuckerung werden die noch vorhandenen Feststoffe, vorwiegend Zellwandbestandteile durch Zentrifugieren oder Filtrieren abgetrennt. Da diese Feststoffe durch den angewandten schonenden Aufschluß der Stärke nicht verändert werden, erfolgt ihre Abtrennung problemlos.

Die nächsten Schritte des erfindungsgemäßen Verfahrens dienen der Abtrennung der noch kolloid- oder molekulardispers enthaltenen Nichtzuckerstoffe, z.B. Farbstoffe, Mineralstoffe, lösliche Stickstoffverbindungen, wofür sich eine Adsorptionsfiltration mit Aktivkohle, gefolgt von einer Ionenaustauscherbehandlung am günstigsten erwies.

Das erfindungsgemäße Verfahren der Direkthydrolyse bietet gegenüber der konventionellen Verfahrensweise noch weitere, entscheidende Vorteile. So können einmal durch den Wegfall der apparatemäßig sehr aufwendigen Stärkeisolierung beim Neubau einer Zuckersirupfabrik ein Großteil der Investitionskosten eingespart werden. Weiters werden bei der großtechnischen Isolierung von Tapiokastärke aus frischen Cassavawurzeln nur etwa 85 bis 90 % der enthaltenen Stärke gewonnen (CARANSA, 1980; MEUSER et al, 1978). Im Gegensatz dazu wird bei der Direktverzuckerung fast die gesamte im Rohstoff vorhandene Stärkemenge genutzt. Insgesamt gesehen ist daher die Glucoseausbeute beim erfindungsgemäßen Verfahren höher.

Ein bedeutendes Anwendungsgebiet des erfindungsgemäßen Verfahrens eröffnet sich bei Einsatz von getrockneten Cassavaprodukten als Rohstoff; denn frische Cassavawurzeln sind nur sehr kurze Zeit lagerfähig und müssen deshalb rasch verarbeitet werden. Außerdem ist die Ernte der Cassavawurzeln nur maximal 6 - 7 Monate im Jahr möglich, weshalb bei Verarbeitung frischer Wurzeln ein Kampagnebetrieb erforderlich ist. Die Dimensionen der notwendigen Anlagen und damit verbunden auch die Investitionskosten sind bei einem Kampagnebetrieb aber größer. Bedenkt man die Verhältnisse in den cassavaproduzierenden Ländern - Produktion durch weitverstreute kleine Bauern, mangelnde Infrastruktur usw. - so ist die Rohstoffversorgung einer größeren Industrieanlage mit frischen Cassavawurzeln mit Problemen verbunden. Anderseits kann die Herstellung getrockneter Cassavaprodukte in einfacher Weise durch den Kleinproduzenten selbst erfolgen (Waschen, Zerkleinern, Sonnentrocknen). Tatsächlich wird ein großer Teil der Cassavaernte in dieser Weise verarbeitet. Die Probleme der Lagerung und des Transportes werden durch die Möglichkeit der Verarbeitung von Trockenprodukten, wie sie das erfindungsgemäße Verfahren bietet, wesentlich erleichtert. Damit kann eine über das ganze Jahr gleichmäßig verteilte Produktion gewährleistet werden und die Anlage kann kleiner dimensioniert werden.

Das erfindungsgemäße Verfahren wird durch folgende Beispiele näher erläutert:

Beispiel 1:

100 kg frische, geschälte Cassavawurzeln wurden mit einem Reibschnitzler (5 mm Siebeinsatz) grob vorzerkleinert, in 100 l Leitungswasser suspendiert und die erhaltene Suspension unmittelbar zur Feinzerkleinerung über eine Zahnkolloidmühle geleitet. Anschließend wurden die Feststoffe mit einem Technikumsdekanter bei 2000 g abgetrennt und der Feststoffkuchen noch im Dekanter mit Leitungswasser ausgewaschen.

Die Feststoffe wurden hierauf kontinuierlich unter Zumischung von alpha-Amylase (0,5 l/t Stärke-TS; Type Termamyl L 120, Fa. Novo) und Verdünnungswasser (Prozeß-bzw. Absüßwasser) in einen zylindrischen Aufschlußbehälter (Inhalt 30 l) gepumpt. Die zugepumpte Menge an Verdünnungswasser wurde so gewählt, daß sich nach Suspension der Feststoffe im Behälter eine Trockensubstanzkonzentration von etwa 20 % ergab. Der Aufschlußbehälter war mit einem Rührer ausgestattet und wurde durch Einleiten von Dampf direkt beheizt. Zur Konstanthaltung der gewünschten Aufschlußtemperatur von 90°C war außerdem eine Temperaturregelung eingebaut. Zu Versuchsbeginn wurden in den Behälter vorerst etwa 10 l Verdünnungswasser vorgelegt, diese auf 90°C aufgeheizt und dann mit dem Einbringen des Rohstoffes begonnen. Gleichzeitig wurde am Behälterboden mit dem Abzug der aufgeschlossenen, verflüssigten Lösung begonnen. Der Zu-und Abfluß wurde so geregelt, daß sich eine mittlere Verweilzeit von 10 min ergab. Die am Boden des Aufschlußbehälters abgezogene Lösung wurde in einem Bottich gesammelt, auf 60°C abgekühlt, der pH-Wert mit verd.$H_2SO_4$ auf 4,5 eingestellt, 0,7 l/t Stärke-TS Amyloglucosidase (AMG L 300, Fa. Novo) zugegeben und bei dieser Temperatur 65 Stunden vollständig verzuckert. Danach wurden die noch vorhandenen Feststoffe mit einem Technikumsdekanter bei 2000 g abgetrennt. In das erhaltene Dekantat wurden 0,5 % Aktivkohle (Typ CG1, Fa. Norit) und 2 % Filterhilfsmittel bez. auf Rohsaft-TS eingerührt und 1 Stunde bei 60°C gerührt. Ein Rahmenfilter wurde mit Filterhilfsmittel vorangeschwemmt und dann die Aktivkohle und die daran adsorbierten Nichtzuckerstoffe aus dem Rohsaft abfiltriert. Im Anschluß daran wurde der gereinigte Rohsaft auf eine Trockensubstanz von 40 % (G/G) unter Vakuum eingedampft.

Zur Abtrennung noch vorhandener Aktivkohleteilchen wurde der Saft über ein Schichtenfilter feinfiltriert. Das klare Filtrat wurde zur Entsalzung vorerst über einen stark sauren Kationenaustauscher in der Wasserstoff-Beladungsform und hierauf unmittelbar über einen mittelbasischen Anionenaustauscher in der Hydroxid-Beladungsform geleitet.

Der erhaltene Glucosesirup wies eine Leitfähigkeit von weniger als 0,01 mS.cm$^{-1}$ auf. Farbstoffe waren nach der ICUMSA-Methode (420 nm) nicht mehr nachweisbar. Der Proteingehalt (Kjeldahl-Methode) und der Aschegehalt (gravimetrisch) lagen ebenfalls unter der Nachweisgrenze. Der mit der HPLC-Methode ermittelte Glucosegehalt betrug 96,01 % i.d.TS und der Fruktosegehalt 3,63 % i.d.TS.

Der Glucosesirup wurde anschließend enzymatisch isomerisiert. Dazu wurden 40 g Glucoseisomerase (Type Sweetzyme Q, Fa. Novo) in eine temperierbare Säule gefüllt und der entgaste Sirup nach Zugabe von 0,2 g/l $MgSO_4.7H_2O$ und pH-Einstellung auf 7,8 darüber geleitet. Die Temperatur in der Säule betrug 57°C und die Durchflußgeschwindigkeit wurde über den optischen Drehwert so gesteuert, daß sich ein konstanter Isomerisierungsgrad von ca. 42 % ergab. Im isomerisierten Saft wurden 22,19 % (G/G) Glucose, 17,06 % (G/G) Fruktose und 0,77 % (G/G) Di- und Oligosaccharide festgestellt.

Die Versuchsbedingungen und erhaltenen Ergebnisse sind übersichtlich in Tabelle 1 angegeben.

Tabelle 1
Cassavawurzeln

| Probe | Versuchs-bedingungen | TS G/G % | Protein % TS | Asche % TS | Farbein-heiten 420 nm | Leit-fähigk. mS | Glucose % TS | Fructose % TS | Stärke % TS |
|---|---|---|---|---|---|---|---|---|---|
| Reibsel (100 kg) | Reibschnitz-ler (5 nm) | 40,23 | 1,47 (100%) | 1,57 (100%) | | | 3,63 | 2,78 | 85,20 |
| Fest-stoffe nach De-kantation | ca. 2000 g | 49,78 | 0,35 (23,8%) | 0,71 (36,9%) | | | | 0,25 | 93,40 |
| nach Ver-flüssigung | bei 90 Grad | 22,37 | | | | | | | |
| nach Ver-zuckerung | 67 Std. | 24,03 | | | | | | | |
| Dekantat nach 2 Dek. | ca.2000g | 23,52 | 0,54 | 0,31 | | | 94,49 | 0,89 | |
| nach Ak-tivkohlentf. | 0,5% CG1/TS | | 0,38 | 0,44 | | 0,90 | | | |
| nach Konz. u. Feinfiltr. | | 43,45 | | | 50 | 0,66 | | | |
| nach Kationen-u. Anionenaus-tauscher | | 43,87 | n.n.*) | n.n.*) | n.n.*) | 0,01 | 96,01 | 3,63 | |

n.n.*) = nicht nachweisbar

5

Beispiel 2:

60 kg Cassavachips wurden mit einer Messermühle auf eine Korngröße unter 2 mm vermahlen, in 125 l Leitungswasser suspendiert und die erhaltene Suspension zur Feinzerkleinerung über eine Zahnkolloidmühle geleitet. Anschließend wurden die Feststoffe mit einem Technikumsdekanter bei 2000 g abgetrennt und der Feststoffkuchen dabei noch im Dekanter mit Leitungswasser ausgewaschen.

Die Feststoffe wurden hierauf kontinuierlich unter Zumischung von alpha-Amylase (0,5 l/t Stärke-TS; Type Termamyl L 120, Fa. Novo) und Verdünnungswasser (Prozeß-bzw. Absüßwasser) in einen zylindrischen Aufschlußbehälter (Inhalt 30 l) gepumpt und, wie bei Beispiel 1 beschrieben, der Aufschluß, die Verflüssigung und die Verzuckerung durchgeführt. Die Reinigung des Hydrolysates erfolgte ebenfalls so, wie in Beispiel 1 angeführt, nur wurden hier 1,0 % Aktivkohle (Typ CG1, Fa. Norit) bez. auf Rohsaft-TS eingesetzt.

Der erhaltene Glucosesirup wies eine Leitfähigkeit von 0,01 mS.cm$^{-1}$ auf. Nach der ICUMSA-Methode wurden bei 420 nm 2 Farbeinheiten gefunden. Der Proteingehalt (Kjeldahl-Methode) und der Aschegehalt (gravimetrisch) lagen unter der Nachweisgrenze. Der mit der HPLC-Methode ermittelte Glucosegehalt betrug 95,7 % i.d.TS und der Fruktosegehalt 2,9 % i.d.TS.

Der Glucosesirup wurde in gleicher Weise wie bei Beispiel 1 isomerisiert. Im isomerisierten Sirup wurden 21,5 % (G/G) Glucose, 16,9 % (G/G) Fruktose und 1,8 % (G/G) Di- und Oligosaccharide festgestellt.

Die Versuchsbedingungen und erhaltenen Ergebnisse sind übersichtlich in Tabelle 2 angegeben.

Tabelle 2
Cassavachips

| Probe | Versuchs-bedingungen | TS G/G % | Protein % TS | Asche % TS | Farbein-heiten 420 nm | Leit-fähigk. mS | Glucose % TS | Fructose % TS | Stärke % TS |
|---|---|---|---|---|---|---|---|---|---|
| Chipsmehl | Messermühle 2 nm | 89,59 | 2,09 (100%) | 2,83 (100%) | | | 1,89 | 0,74 | 80,98 |
| Feststoffe nach De-kantation | ca.2000g | 51,14 | 0,84 (40,2%) | 1,12 (39,6%) | | | | | |
| nach Ver-flüssigung | bei 90 Grad | 22,27 | | | | | | | |
| nach Ver-zuckerung | 66 Std. | 27,30 | | | | | | | |
| Dekantat nach 2 Dek. | ca.2000g | 26,91 | 0,44 | 0,49 | | | | | |
| nach Ak-tivkohlentf. | 1,0% CG1/TS | | | | | | 96,01 | 0,35 | |
| nach Konz. u. Feinfiltr. | | 42,46 | 0,22 | 0,45 | 369 | 0,80 | | | |
| nach Kationen- u. Anionenaus-tauscher | | 42,84 | n.n.*) | n.n.*) | 2 | 0,01 | 95,69 | 2,86 | |

*) n.n. = nicht nachweisbar

7

**Patentansprüche**

1. Verfahren zur Herstellung von Zuckersirupen aus stärkehältigen Rohstoffen, insbesondere Cassava (Maniok), gekennzeichnet durch die Kombination der Maßnahmen, daß

a) aus zerkleinertem Rohstoff wasserlösliche Inhaltsstoffe ausgewaschen werden;

b) die im Rohstoff enthaltene Stärke ohne Isolierung unter schonenden Bedingungen, d.h. bei Temperaturen unter 100°C, vorzugsweise bei 85 - 95°C, und ohne weiteren mechanischen Aufschluß, und bei Gehalten an Trockensubstanz von über 20 % kontinuierlich verkleistert, enzymatisch mit alpha-Amylase verflüssigt und danach mit Glucoamylase verzuckert wird;

c) die Feststoffe aus der verzuckerten Maische durch Zentrifugieren oder Filtrieren abgetrennt werden;

d) der gewonnene Saft durch Adsorptionsfiltration mit Aktivkohle gereinigt und

e) durch Behandlung mit einer Kombination eines Kationenaustauschers und eines Anionenaustauschers entsalzt wird.

2. Verfahren nach Anspruch 1, dadurch gekennzeichnet, daß als Rohstoff frische Cassavawurzeln verwendet werden, welche vorzugsweise grob vorzerkleinert und anschließend naßfein zerkleinert werden.

3. Verfahren nach Anspruch 1, dadurch gekennzeichnet, daß als Rohstoff getrocknete Cassavaprodukte in Form von Chips oder Pellets verwendet werden, die vorzugsweise grob trocken vorgemahlen und anschließend naßfein zerkleinert werden.

4. Verfahren nach Anspruch 1, dadurch gekennzeichnet, daß das bei der Feststoffabtrennung anfallende Waschwasser als Verdünnungswasser beim Stärkeaufschluß eingesetzt wird.

5. Verfahren nach Anspruch 1, dadurch gekennzeichnet, daß zur Entsalzung des Saftes zuerst ein stark saurer Kationenaustauscher verwendet wird.

6. Verfahren nach den Ansprüchen 1 und 5, dadurch gekennzeichnet, daß der Saft nach Behandlung mit dem sauren Kationenaustauscher mit einem mittel- bis starkbasischen Anionenaustauscher behandelt wird.

**Claims**

1. A process for producing sugar sirups from amylaceous raw materials, such as cassava (manioc), characterized by the combination of the following measures:

a) washing water-soluble ingredients out of crushed raw materials;

b) continuously gelatinizing under careful conditions, i.e., at temperatures of below 100°C, preferably 85-95°C, and without further mechanical disintegration, and at dry substance contents of above 20 %, enzymatically liquefying with alpha-amylase and then saccharifying with glucoamylase, the starch contained in the raw materials without isolation thereof;

c) separating the solids from the saccharified mash by centrifugation or filtration;

d) purifying the juice obtained by adsorption filtering with activated carbon; and

e) desalting the same by treatment with a combination of a cation exchanger and an anion exchanger.

2. A process according to claim 1, characterized in that fresh cassava roots are used as raw materials, which preferably are coarsely crushed at first and are comminuted wet thereafter.

3. A process according to claim 1, characterized in that dried cassava products in the form of chips or pellets are used as raw materials, which preferably are coarsely ground dry at first and are comminuted wet thereafter.

4. A process according to claim 1, characterized in that the washings incurring at the separation of solids are used as diluent water at the hydrolysis of starch.

5. A process according to claim 1, characterized in that a stongly acid cation exchanger is, at first, used for desalting the juice.

EP 0 277 934 B1

**6.** A process according to claims 1 and 5, characterized in that the juice, after treatment with said acid cation exchanger, is treated with a medium to strongly basic anion exchanger.

**Revendications**

**1.** Procédé pour la production de sirops de sucre à partir de matières bruts amylacés, notamment de la cassava (manioc), caractérisé par la combinaison des mesures de
    a) laver des ingrédients solubles dans l'eau des matières bruts concassés;
    b) gélatinifier continuellement, sans isolation, dans des conditions soigneux, c'est-à-dire aux températures au-dessous de 100 °C, de préférence à 85-95 °C, et sans disintégration mécanique additionelle ainsi qu'aux teneurs de matières sèches de plus de 20 %, liquéfier par voie enzymatique au moyen de l'alpha-amylase, puis saccharifier avec de la glucoamylase, l'amidon contenu dans les matières bruts;
    c) séparer par centrifugation ou filtration les solides de la trempe saccharifiée;
    d) purifier par filtration d'adsorption avec du charbon actif le jus obtenu; et
    e) le dessaler au moyen d'une combinaison d'un échangeur de cations et un échangeur d'anions.

**2.** Procédé selon la revendication 1, caractérisé en ce que l'on utilise comme matières bruts des racines de cassava fraiches, qui, de préférence, sont concassées préalablement puis disintégrées à l'état humide.

**3.** Procédé selon la revendication 1, caractérisé en ce que l'on utilise comme matières bruts des produits de cassava séchés en forme de chips ou de pellets, qui, de préférence, sont concassés préalablement à l'état sec puis disintégrées à l'état humide.

**4.** Procédé selon la revendication 1, caractérisé en ce que les petites eaux obtenues lors de la séparation des solides sont utilisées en tant qu'eaux de dilution pour l'hydrolysation de l'amidon.

**5.** Procédé selon la revendication 1, caractérisé en ce que l'on utilise d'abord un échangeur de cations fortement acide pour dessaler le jus.

**6.** Procédé selon les revendications 1 et 5, caractérisé en ce que le jus, après le traitement avec l'échangeur de cations fortement acide, est traité avec un échangeur d'anions moyennement à fortement basique.

9